# EUROPEAN PATENT SPECIFICATION

(11) **EP 3 946 177 B1**
(45) Date of publication and mention of the grant of the patent: **18.03.2026**
(21) Application number: 20719562.9
(22) Date of filing: 30.03.2020
(51) Int. Cl.: A61F 5/443, A61F 5/449

(54) **A PLIABLE SKIN BARRIER RING**
BIEGSAMER HAUTBARRIERERING
ANNEAU DE BARRIÈRE CUTANÉE PLIABLE

(30) Priority: 01.04.2019 DK PA201970196
(43) Date of publication of application: 09.02.2022
(73) Proprietor: Coloplast A/S, 3050 Humlebaek (DK)
(72) Inventor: HECKLER, Ilona Maria, 2990 Nivaa (DK)
(86) International application number: PCT/DK2020/050081
(87) International publication number: WO 2020/200382

(56) References cited:
- EP-A1- 0 686 381
- EP-A1- 3 253 342
- EP-A1- 3 270 835
- EP-B1- 0 686 381
- EP-B1- 3 253 342
- EP-B1- 3 270 835
- WO-A1-2016/124202
- US-A1- 2008 097 361
- US-A1- 2018 116 859

## Description

### Background

Stomal output often contains body fluids and visceral contents that are aggressive to both the skin of a user and to ostomy devices, in particular these have a detrimental effect on the efficiency and integrity of the adhesive materials that are applied to attach the ostomy device to the user's skin surface.

A number of ostomists develop so-called retracted or recessed stomas. Due to inevitable post-surgical and ongoing physiological changes of the ostomist's body, the stoma may from the outset or over time sink or retract into the abdomen creating a recess in the body surface where the stoma is placed. Applying a standard planar base plate around a retracted stoma would, for one thing, leave the area around the stoma uncovered (gap between the peristomal skin surface and adhesive surface of base plate) and thereby exposed to the output from the stoma. Moreover, in some cases the stoma may be retracted to such a degree that it cannot extend through the through-going hole in the base plate thus resulting in frequent leakage problems. In order to address the issue of retracted stomas, and the encountered frequent leakages, convex base plates have been developed.

Traditionally, convex base plates have been made by providing a relatively stiff pre-cast or moulded unit of a convex shape of form and attaching it to one side of an adhesive wafer whereby the base plate obtains a convexity. Such products have been available on the markets for many years.

The primary purposes and effects of these convex base plates are that they are able to withhold the peristomal skin of the ostomist's body from collapsing (e.g. due to excess skin folds) and to increase the ability of the stoma to protrude adequately past the interface between the base plate and the body surface due to the reactive force provided by them and thus deposit the stomal output directly into a collecting bag for human body wastes. Thereby the risk of leakages is reduced since stomal output is less prone to end up underneath the adhesive surface of the base plate where it may attack and eventually disintegrate the adhesive seal.

Generally, however, since these products are relatively stiff and inflexible, they will not follow the movements of the user's body caused by physical activity very well. More severely, experience has shown that the use of these products may in some cases result in peristomal skin damage such as pressure wound ulceration, bruises and/or general skin irritation.

Moreover, the pre-cast or moulded unit of these convex base plates cannot be fitted to abutment with the stoma or even provided close to the stoma. Instead, they require some radial clearance between the innermost edge of the convex pre-cast or moulded unit and the stoma's surface.

This is due to several reasons: first of all, experience has shown that if a stiff unit is in direct contact with the mucus membrane of a stoma's surface, it tends to cut and irritate it, which may eventually cause the stoma to bleed and incur serious complications. Also, the pre-cast or moulded unit may prevent the stoma from expanding freely, the expansion(s) caused by the peristaltic movements of the intestine.

In addition, the presence of such a radial clearance between the innermost edge of the pre-cast or moulded unit and the stoma-receiving through-going hole means that there will be a lack of adequate constant pressure against the skin surface, particularly in the immediate peristomal skin area. This lack of adequate pressure often means that the immediate peristomal skin of the user, between the innermost edge of the convex unit and the stoma's surface, is distanced axially in relation to the skin otherwise forced in position by the convex unit. This phenomenon increases the risk of leakages.

Moreover, almost all users need to perform some sort of customizing action to make a fresh appliance fit as precisely as possible to the shape of their stoma's surface (contour(s)) to provide the best possible security against leakages. To that end, almost every ostomy appliance, including those having a pre-cast or moulded convex unit, has a possibility to cut (e.g. with scissors) in the surface of the base plate to make it fit as good as possible. This is often facilitated by means of a small hole, referred to as a pre-cut hole or "starter-hole" provided in the center of the base plate at manufacture.

With regard to the traditional convex base plates, the presence of the pre-cast or moulded stiff convex unit sets a limit to the level of customization possible, in terms of the hole size and shape in such products, as it is not possible to cut the pre-cast convex unit.

One example of such a traditional and relatively stiff convex base plate is disclosed in EP748195.

In more recent years an increased variety of convex base plate products have been made available to ostomists. As every user has individual needs due to his or her unique body and stoma shape, experience has shown that the known stiff convex products fail to overcome each and every kind of those needs. Particularly, if the recess in the body is relatively shallow, i.e. when the stoma is only retracted to a relatively small degree, a less bulky and less stiff convex base plate may be appropriate. One example of such a convex base plate is provided by the disclosure of EP2497449.

Another example is EP 0.686.381 which discloses an ostomy appliance comprising a collection pouch and faceplate assembly. The faceplate assembly includes a flexible patch having a stoma-receiving opening, a first layer of skin-friendly hydrocolloid-containing adhesive material along one side of the patch about the opening for securing the faceplate assembly to peristomal skin surfaces, and a second layer of relatively soft, easily-deformable and extrudable, adhesive sealant material of a composition that is resistant to being dissolved or disintegrated by stomal fluids and that immediately surrounds the opening. The second layer is displaceable inwardly and axially into the opening for forming a stoma-engaging annular gasket to prevent stomal fluids from contacting the first adhesive layer.

Moreover, many users also develop a hernia resulting from the ostomy surgery. For some, this means that the position on the abdominal skin surface where the stoma is located is on the hernia, i.e. on a bulking, protruding outward body profile. Like any other stoma, a stoma on a hernia can be retracted or recessed and it can be very difficult to provide a proper seal around a recessed stoma on a hernia or other outwardly protruding body profile.

Ostomists and health care professionals alike would welcome improvements in ostomy devices to better accommodate this type of body profiles.

### Summary

The present disclosure provides aspects of a pliable skin barrier ring adapted for placement in a skin fold or crease located around a stoma of a user according to the appended claims.

### Brief Description of the Drawings

The accompanying drawings are included to provide a further understanding of embodiments and are incorporated in and constitute a part of this specification. The figures illustrate embodiments and together with the description serve to explain principles of embodiments. Other embodiments and many of the intended advantages of embodiments will be readily appreciated as they become better understood by reference to the following detailed description. Like reference numerals designate corresponding similar parts.

Various exemplary embodiments and details are described hereinafter with reference to the figures when relevant. It should be noted that the figures may or may not be drawn to scale and that elements of similar structures or functions are represented by like reference numerals throughout the figures. It should also be noted that the figures are only intended to facilitate the description of the embodiments. They are not intended as an exhaustive description of the invention or as a limitation on the scope of the invention. In addition, an illustrated embodiment needs not have all the aspects or advantages shown. An aspect or an advantage described in conjunction with a particular embodiment is not necessarily limited to that embodiment and may be practiced in any other embodiments even if not so illustrated, or if not so explicitly described.
Figure 1 is a cross-sectional, perspective view of one embodiment of a pliable barrier ring of the disclosure.
Figure 2 is an enlarged cross-sectional, perspective partial view of one embodiment of a pliable barrier ring of the disclosure.
Figure 3 is an enlarged cross-sectional, perspective partial view of one embodiment of a pliable barrier ring of the disclosure.
Figure 4 is a schematic, cross-sectional view of one embodiment of a pliable barrier ring of the disclosure.
Figure 5 is a schematic, cross-sectional view of one embodiment of a pliable barrier ring of the disclosure.
Figure 6A is a perspective view of one embodiment of a pliable barrier ring of the disclosure showing the ring being stretched.
Figure 6B is a top view of one embodiment of a pliable barrier ring of the disclosure.
Figure 6C is a top view of the embodiment of the pliable barrier ring of Figure 6B in a stretched condition.
Figure 7 is a perspective view of one embodiment of a pliable barrier ring of the disclosure showing the ring being compressed.

### Detailed Description

In the following Detailed Description, reference is made to the accompanying drawings, which form a part hereof, and in which is shown by way of illustration specific embodiments in which the invention may be practiced. In this regard, directional terminology, such as "top," "bottom," "front," "back," "leading," "trailing," etc., is used with reference to the orientation of the Figure(s) being described. Because components of embodiments can be positioned in a number of different orientations, the directional terminology is used for purposes of illustration and is in no way limiting. It is to be understood that other embodiments may be utilized, and structural or logical changes may be made without departing from the scope of the present invention. The following detailed description, therefore, is not to be taken in a limiting sense, and the scope of the present invention is defined by the appended claims.

It is to be understood that the features of the various exemplary embodiments described herein may be combined with each other unless specifically noted otherwise.

Throughout this disclosure, the words "stoma" and "ostomy" are used to denote a surgically created opening bypassing the intestines or urinary tract system of a person. The words are used interchangeably, and no differentiated meaning is intended. The same applies for any words or phrases derived from these, e.g. "stomal", "ostomies" etc. Also, the solid and liquid wastes emanating from the stoma may be referred to as both stomal "output," "waste(s)," and "fluids" interchangeably. A subject having undergone ostomy surgery may be referred to as "ostomist" or "ostomate" - moreover, also as "patient" or "user". However, in some cases "user" may also relate or refer to a health care professional (HCP), such as a surgeon or an ostomy care nurse or others. In those cases, it will either be explicitly stated or be implicit from the context that the "user" is not the "patient" him- or herself.

Throughout this disclosure, the words "mouldable" and "mouldability" are used to denote the process of changing the shape of the adhesives by stretching or compressing. The words can be used interchangeably with the words "shapeable" and shapeability", and no differentiated meaning is intended.

In the following, whenever referring to proximal side of a device or part of a device, the referral is to the skin-facing side, when the ostomy appliance is worn by a user. Likewise, whenever referring to the distal side of a device or part of a device, the referral is to the side facing away from the skin, when the ostomy appliance is worn by a user. In other words, the proximal side is the side closest to the user, when the appliance is fitted on a user and the distal side is the opposite side - the side furthest away from the user in use.

The axial direction is defined as the direction of the stoma, when the appliance is worn by a user. Thus, the axial direction is generally perpendicular to the skin or abdominal surface of the user.

The radial direction is defined as transverse to the axial direction that is transversely to the direction of the stoma. In some sentences, the words "inner" and "outer" may be used. These qualifiers should generally be perceived with reference to the radial direction, such that a reference to an "outer" element means that the element is farther away from a centre portion of the ostomy appliance than an element referenced as "inner". In addition, "innermost" should be interpreted as the portion of a component forming a centre of the component and/or being adjacent to the centre of the component. In analogy, "outermost" should be interpreted as a portion of a component forming an outer edge or outer contour of a component and/or being adjacent to that outer edge or outer contour.

"Convex" is intended to define that an element or its corresponding surface has a shape or form that provides an overall convexity. In other words, while a smaller section or zone making up a portion of the overall element or its corresponding surface can have e.g. a linear shape or form, the element or surface as a whole has a convex shape. It is further to be understood that if for a sheet- or plate-like element one major surface has a convex shape, the opposite major surface may be seen as having a corresponding concave shape.

"Release liner" is intended to define a liner or lining material covering the adhesive material and ensuring that the properties of the adhesive material are preserved, and that the adhesive surface is not laid open until just before the use.

The use of the phrases "substantial" or "substantially" as a qualifier to certain features or effects in this disclosure is intended to simply mean that any deviations are within tolerances that would normally be expected by the skilled person in the relevant field.

The use of the word "generally" as a qualifier to certain features or effects in this disclosure is intended to mean - for a structural feature: that a majority or major portion of such feature exhibits the characteristic in question, and - for a functional feature or an effect: that a majority of outcomes involving the characteristic provide the effect, but that exceptionally outcomes do no provide the effect.

The use of the phrases "approximate" or "approximately" as a qualifier to certain features or effects in this disclosure is intended to simply mean that a value may not be exactly correct, but fairly close to correct and within variations that would normally be expected by the skilled person in the relevant field.

The present invention relates to a pliable skin barrier ring as set out in the claims.

According to the disclosure, the pliable barrier ring is mouldable, meaning that it can be shaped by the user applying finger pressure to the pliable barrier ring. Thereby, the pliable barrier ring can be shaped by a user such as to wholly or partially fit into skin folds, scars or creases around a stoma. As a further advantage, the pliable barrier ring can also be shaped to closely match the shape of a given stoma, i.e. to generally "follow" a contour of the external surface of the stoma. Moreover, by providing more than one type of mouldable adhesive material (i.e. differentiating adhesive materials for the provision of a barrier ring), further features can be attributed to the pliable barrier ring. All such options can aid in helping to provide a tight seal around the stoma. In embodiments, the first relatively harder adhesive material is a pressure sensitive adhesive material. In embodiments, the second relatively softer adhesive material is a pressure sensitive adhesive material. In embodiments, both the first relatively harder adhesive material and the second relatively softer adhesive material are pressure sensitive adhesive materials. In embodiments, the first relatively harder adhesive material and/or the second relatively softer adhesive material includes a silicone-based adhesive material.

According to the disclosure, both the first adhesive material and the second adhesive material of the pliable barrier ring are to some degree mouldable. However, the first adhesive material forming the bulk portion of the barrier ring is of a nature which is less mouldable than the second adhesive material. This provides for the first adhesive material to be more dimensionally stable than the second adhesive material. That higher degree of dimensional stability in turn helps provide for the bulk portion of the first adhesive material to incur and maintain the proximally projecting expanded shape to the central portion of the barrier ring. In other words, the first adhesive material in the bulk portion provides a portion of the barrier ring extending proximally away from the flange portion of the barrier ring, thereby incurring what is sometimes referred to in the art as convexity. According to the disclosure, the barrier ring can be considered a pliable, convex barrier ring, wherein the first adhesive material contributes primarily, but not exclusively, to providing the proximally projecting extended shape (convexity) to the barrier ring while still exhibiting a plastic deformation capability, and wherein the second adhesive material contributes primarily to mouldability and adaptability of the pliable barrier ring and only little or not at all to the dimensional stability of the barrier ring.

A further advantage of the higher degree of dimensional stability of the first adhesive material is that the flange portion of the first layer of adhesive material also provides for increased stability of the outermost peripheral edge of the pliable barrier ring, in particular it provides for avoidance of a roll-up (or "curl-up") tendency of the outermost peripheral edge subsequent to removal of any release liners from the proximal and distal surfaces of the pliable barrier ring. From experience it is known that relatively thin (flange) portions of adhesive materials having some degree of elastic characteristics (i.e. in the framework of the present disclosure corresponding to a tan(δ) below 1) tends to roll up when the one or more release liners supporting the thin portion is removed. Due to the two-layer construction of the barrier ring disclosed herein, including the first layer of relatively harder adhesive material, the present barrier ring is less prone to be subject to such rolling up subsequent to removal of the release liners on the proximal and distal surface of the barrier ring.

To meet the above identified and other characteristics, the first relatively harder adhesive material suitably comprises a polyisobutylene (PIB), such as Oppanol B12 SFN from BASF; a Styrene/Isoprene/Styrene (SIS), such as Kraton^{®} D1161-BT from Kraton Polymers Nederland; Butyle rubber 101-3 from Arlanxeo; a resin, such as Arkon p90 from Caldic Danmark A/S; and various absorbent components including a HEC, such as Natrosol 250 HX from Ashland Speciality Ingredients; a LM ester such as Pectin LM 12 CG-Z/200 from CP Kelco APS; a CMC such as Akucell AF2881 from AkzoNobel; and gelatine from PB Leiner.

To meet the above identified and other characteristics, the second relatively softer adhesive material suitably includes, but is not limited to, adhesive materials comprising an adhesive polymer component selected from the group consisting of a mixture of a polyethylene copolymer and polypropylene glycol (PPG), and styrene-ethylene/butylene-styrene (SEBS) block copolymer; the adhesive material further including polyolefin, polybutene, polyacrylic acid (PAA), and at least one further absorbent material. Suitable examples of such adhesives are disclosed and claimed in applicant's international application WO 2017/032381.

In embodiments according to the disclosure, the pliable skin barrier ring is adapted for placement in a skin fold or crease located around a stoma of a user. The pliable barrier ring can be used to provide a seal around the stoma and can be used in combination with a regular ostomy device comprising a collecting pouch and an adhesive wafer. In such a situation, the user may start by fitting the pliable barrier ring closely around the stoma and will then apply the adhesive wafer of the ostomy device on top of or immediately next to the pliable barrier ring. Alternatively, the user may start by applying the pliable barrier ring to the adhesive wafer of the ostomy device before attaching the combined device to the skin surrounding the stoma. In either way, a tight and secure seal can be provided around the stoma, thereby protecting against leakage.

Particularly, in embodiments, the relatively softer second adhesive material is configured to start flowing at a lower temperature than the relatively harder first adhesive material. This provides for the second adhesive material to be able to deform or flow into the skin folds or creases around the stoma before the first adhesive material begins to flow. Thus, in embodiments the layer of the second adhesive material helps provide for a secure and tight fit of the pliable barrier ring to the skin surface surrounding the stoma (the peristomal skin area). In embodiments, the second adhesive material is configured to flow into the skin surface at a temperature of approximately 32°C (normal skin surface temperature). In embodiments, the first adhesive material is configured to flow at the same rate as the second adhesive material at a temperature that is higher than 32°C. In embodiments, the first relatively harder adhesive material layer flows at a slower rate than the second relatively softer adhesive material layer. In embodiments, the relatively softer second adhesive material layer is configured to have a higher tack than the relatively harder first adhesive material layer. In embodiments, the relatively softer second adhesive material layer maintains a high tack even after having been subjected to being moulded.

The pliable skin barrier ring includes a stoma-receiving opening which extends through a central portion of the ring. The central portion of the barrier ring includes at least the proximally projecting expanded shape provided by the bulk portion of the first layer of adhesive material, the portion of the layer of the second adhesive material forming the proximal surface of the ring on the expanded shape and the stoma-receiving opening. In embodiments, the stoma-receiving opening is centred in the central portion of the ring. In other embodiments, the stoma-receiving opening can be provided in a radially offset location of the central portion of the ring. In embodiments, a diameter of the stoma-receiving opening is chosen from the range of 10-60 mm, such as 10-45 mm, such as 15-40 mm.

The first adhesive material and the second adhesive material of the pliable barrier ring each further possess some absorption capacity, meaning that each of the adhesives can absorb some moisture or liquid from the surroundings. This allows the pliable barrier ring to absorb moisture from the skin around the stoma (e.g. sweat), which helps keep the skin surface dry, thereby preventing damage to the skin (skin maceration).

For preparing the pliable skin barrier ring for use, any release liners are first removed, and the ring is placed on the skin surrounding the stoma. The pliable skin barrier ring can then be moulded into shape, if necessary, by pressing it with the fingers to make the ring fit closely in skin folds or creases around the stoma. The pliable ring can also be moulded into closely fitting to an outer contour of the stoma. When the pliable barrier ring has been secured around the stoma, an ostomy device, typically including an adhesive wafer and a collecting bag, can be attached to the skin and/or the distal surface of the pliable barrier ring. After use, the pliable barrier ring is removed from the skin, optionally together with the ostomy device, and discarded. Alternatively, the ostomy device and the pliable barrier ring are attached to each other before being attached to the skin around the stoma, meaning that the user first removes the release liner from the proximal skin facing surface of the base plate and then attaches the distal surface of the pliable skin barrier ring to the proximal surface of the base plate, taking due account of aligning the stoma-receiving openings of the base plate and the barrier ring. Subsequently, the combined (attached together) base plate and barrier ring are applied to the user's skin surface around the stoma.

In embodiments, the second layer of relatively softer adhesive material forms an entirety of an inner peripheral surface of the of the ring at the stoma-receiving opening. It is to be understood that the inner peripheral surface of the ring at the stoma-receiving opening forms the interface between the barrier ring and the stoma-receiving opening. In embodiments, the inner peripheral surface annularly surrounds the stoma-receiving opening. In embodiments, the second layer of the relatively softer adhesive material provides a "lining" of the entire extent of the inner peripheral surface of the ring at the stoma-receiving opening. In embodiments, the second layer of adhesive material is configured to extend in a continuous manner (i.e. unbroken or undivided) over a transition between the proximal surface of the barrier ring and the inner peripheral surface of the ring at the stoma-receiving opening, both surfaces formed by the second layer of relatively softer adhesive material. In embodiments, the transition includes an edge where a plane of the proximal surface of the barrier ring at the central portion meets a tangent to the inner peripheral surface of the ring at the stoma-receiving opening in a substantially orthogonal manner. These embodiments are advantageous *inter alia* in that a two-adhesive-layer barrier ring according to the disclosure can be easily controlled during manufacture, because the relatively softer second adhesive material sits around a major portion of the external surface of the relatively harder, more dimensionally stable first adhesive material, and this configuration in its entirety provides a barrier ring with an overall increased stability compared to single adhesive-layer mouldable barrier rings. Moreover, the two-adhesive-layer barrier ring of this disclosure further provides for reducing the number of necessary components and/or accessories for better creating a leakage-safe seal around the stoma. The barrier ring provides both the necessary structural pressure-incurring capability (the relatively harder first adhesive material) and the mouldability for fitting the adhesive well into the skin creases in the peristomal skin area (the relatively softer second adhesive material). Thus, the user does not have to handle both a stiff, pressure-incurring component and a softer, pliable adhesive material such as a paste adhesive to achieve a good seal of the ostomy appliance around the stoma.

In embodiments, the proximal surface of the barrier ring describes a curve approximating an S-shape, when seen in a cross-sectional view through the ring. The approximating S-shape of the proximal surface of the barrier ring provides for a relatively smooth (or "gentle" or "soft") transition between the flange portion and the bulk portion of the first layer of relatively harder adhesive material. This in turn helps provide the "convexity" or a convex shape of the barrier ring.

In embodiments, a first portion of the proximal surface of the ring provided on the flange portion of the first adhesive layer lies in a first plane, and a second portion of the proximal surface of the ring provided on the bulk portion of the first adhesive layer lies in a second plane. The second plane is parallel to the first plane.

In embodiments, a linear distance between the first plane and the second plane measured along a normal to the first or the second plane is in the range of 5-20 mm. In embodiments, the linear distance is in a range of 5-15 mm, such as 5-13 mm, such as 5-10 mm, such as approximately 8 mm. This range, and any specific value of the linear distance in the range, provides a suitable convexity of the pliable barrier ring. In embodiments, an internal diameter of the stoma-receiving opening of the barrier ring is in the range of 15-50 mm, such as 20-45 mm, such as 25-40 mm, such as 30-35 mm. In embodiments, an overall diameter of the barrier ring (at the outer peripheral edge) is in the range of 50-100 mm, such as 60-85 mm, such as 65-80 mm, such as 70-75 mm. In embodiments, a difference between a first diameter of the barrier ring at the outer peripheral edge and a second diameter of the barrier ring at an inner peripheral edge (parallel with the inner peripheral surface) is approximately 40 mm. In embodiments, a radial width of the barrier ring measured from the inner peripheral edge to the outer peripheral edge is approximately 20 mm, such as exactly 20 mm.

In embodiments, the barrier ring includes an outer planar proximal surface extending in a first plane perpendicular to a centre axis C - C of the stoma-receiving opening, the outer planar proximal surface extending radially inwards from an outermost peripheral edge of the barrier ring and transitioning into an intermediate slanting surface, where the intermediate slanting surface extends radially inwards from the outer planar proximal surface towards an inner planar proximal surface, where the inner planar proximal surface extends in a second plane perpendicular to the centre axis C - C of the stoma-receiving opening and extends radially from the intermediate slanting surface towards an innermost peripheral edge of the barrier ring defining the stoma-receiving opening. In embodiments, the outer planar proximal surface of the barrier ring is formed by the second layer of relatively softer adhesive material at a portion of the barrier ring defined by the flange portion of the first relatively harder adhesive material. In embodiments, the inner planar proximal surface of the barrier ring is formed by the second layer of relatively softer adhesive material at a portion of the barrier ring defined by the bulk portion of the first relatively harder adhesive material.

As can be understood, in other words, the outer planar proximal surface is axially displaced along the axis C - C of the stoma-receiving opening in respect to the inner planar proximal surface. A first radius from the axis C - C of the stoma-receiving opening to an outermost edge portion of the inner planar proximal surface is smaller than a second radius from the axis C - C of the stoma-receiving opening to an innermost edge portion of the outer planar proximal surface, and the inner planar proximal surface is connected to the outer planar proximal surface by the intermediate slanting surface.

In accordance with the disclosure, one objective of configuring the pliable barrier ring to have a proximally projecting expanded shape at the central portion of the ring and thereby incur a convexity to the ring is to assist in making the user's stoma protrude sufficiently from the skin surface. The proximally projecting expanded shape at the central portion is designed to provide the required pressure to the peristomal skin area (immediately around the stoma) to make the stoma able to extend properly into a collecting bag for the stomal output. The pressure is obtained when the barrier ring is provided on or attached to an adhesive base plate of an ostomy appliance, which in turn is adhered to the user's abdominal skin around the stomal area, thereby creating a pressure to the peristomal area from the proximally projecting expanded shape of the central portion of the ring.

In accordance with the disclosure, another objective fulfilled by the inventive pliable barrier ring as discussed herein, is to provide a particularly versatile skin barrier ring in terms of capability to be adapted in shape and size to a user's individual peristomal area and stoma. Unlike currently available barrier ring options, the pliable ring according to the disclosure provides a ring which is mouldable to facilitate a very close (and hence leakage tight) fit to the peristomal area and stoma, yet still also with the capability of offering a high degree of peristomal pressure to make the stoma satisfactorily protrude, even without including a relatively stiffer, thermoplastic element in the barrier ring as otherwise usually seen in the art.

Further advantages of the disclosed barrier ring include the provision of a barrier ring which is more compliant with the user's movements, is less prone to cause pressure wounds in the peristomal area and which may even be cut into smaller segments (portions) by the user (which is further facilitated according to the disclosure because there is no thermoplastic element in the barrier ring). Many users have very uneven and/or irregular peristomal skin surfaces e.g. from surgery scarring, skin maceration etc. For these and other users, the option of cutting the pliable barrier ring of the disclosure into smaller segments is particularly advantageous. It may be that only a portion of the peristomal area requires to be put under pressure for adequate stomal protrusion, and/or only a portion of the peristomal area would benefit from being "filled" with the material of the pliable barrier ring. With the disclosed barrier ring the user has the option of cutting a smaller segment from the barrier ring, e.g. in a shape such as a crescent portion or a quarter portion of the entire ring, and then to attach this segment/portion of barrier ring to the base plate of an ostomy appliance, before applying it around the stoma for use. In this manner, the disclosed barrier ring provides a very versatile accessory for an ostomy appliance, which can easily and intuitively be adapted to create a very close fit to the peristomal skin surface exactly where (and potentially only where) it is needed. This in turn provides a more comfortable solution for the user. Overall, this provides a solution which helps to further reduce the risk of leakage of stomal output.

In a further advantageous implementation, the pliable barrier ring of the disclosure is particularly suitable for users who have developed a hernia, and where the stoma's position on the abdominal skin surface is located on the hernia, i.e. on a bulking, protruding outward body profile. For many of these users, the problem of a retracted (non-protruding) stoma is frequent. Even though the hernia itself is outwardly protruding, the stoma very often "sits" in a hollowed (recessed) peristomal skin portion on the hernia, which makes it difficult for these users to find an ostomy appliance capable of meeting all the requirements for creating a close fit of the appliance around their stoma. The pliable skin barrier ring of the disclosure provides a solution which is very versatile and adaptable and therefore meets the individual user's need for easy customization while simultaneously being able to provide adequate pressure to the peristomal area to make the stoma protrude. Particularly, if the pliable barrier ring of the disclosure is used in combination with a concave ostomy base plate, for example concave base plates as disclosed in applicant's other patent applications published as WO 2016/146135 or WO 2017/041807, users with a hernia are offered a combined option that is better capable of meeting their needs.

In embodiments, the second layer of relatively softer adhesive material is configured to have a substantially constant thickness measured in an axial direction of the ring. This means that the thickness of the second layer of adhesive material is not configured to vary over the extent of the second layer of adhesive material. In embodiments, the portion of the second layer of relatively softer adhesive material forming an entirety of the inner peripheral surface of the ring at the stoma-receiving opening has the same substantially constant thickness as the layer of the second adhesive material forming the proximal surface of the ring. One advantage is facilitation of manufacturing of the pliable barrier ring.

In embodiments, the substantially constant thickness of the second layer of adhesive material is selected from a range of 0.5 - 10.0 mm. In embodiments, the substantially constant thickness of the second layer of adhesive material is selected from a range of 0.5 - 2.0 mm. In embodiments, the thickness is selected to be 0.5 mm. In embodiments, the thickness is selected to be 1.0 mm. In embodiments, the thickness is selected to be 1.5 mm. In embodiments, the thickness is selected to be 2.0 mm. In embodiments, the thickness is selected to be 5.0 mm. In embodiments, the thickness is selected to be 10.0 mm.

In embodiments, a thickness of the flange portion of the first layer of relatively harder adhesive material measured in an axial direction of the ring is in a range of 0.2 - 3.0 mm.

In embodiments, a maximum thickness of the bulk portion of the first layer of relatively harder adhesive material measured in an axial direction of the ring is in a range of 3 - 12 mm, such as 3 - 10 mm.

In embodiments, the relatively harder material of the first adhesive layer has a tangent delta (tan δ) below 0.6 at 1 Hz and a |G*| below 1 MPa in a frequency range of 0.03 - 1.0 Hz with tangent delta at 0.03 Hz being above tangent delta at 1.0 Hz. The values are obtained by subjecting the adhesive materials used for the first and second layers of the pliable barrier ring to a DMA analysis (Dynamic Mechanical Analysis, also known as dynamic mechanical spectroscopy). The test conditions for the DMA analysis are further described below.

In embodiments, the second relatively softer adhesive material layer can be displaced on the proximal surface of the first layer of relatively harder adhesive material without simultaneously deforming the proximal surface of the first layer of relatively harder adhesive material. In other words, the second softer adhesive material can be pressed or nudged in relation to the relatively harder first adhesive material using finger pressure, and thereby the second softer adhesive material can shift position on the proximal surface of first layer of relatively harder adhesive material. In this manner, a local thinning or thickening and/or redistribution of the second adhesive material effected by the user can be achieved. Thus, in turn the barrier ring, in particular a contour of the proximal surface thereof, can be manipulated ("plied") and hence customized into an individual user's needs. Also, the pliable barrier ring will be softer and smoother to manipulate and will more easily flow into the skin surface because of the two-layer construction, by which the second relatively softer adhesive material helps provide for a more pliable construction.

In embodiments, the second relatively softer adhesive material layer and the first layer of relatively harder adhesive material are configured to be co-deformable by a user's finger pressure. Being co-deformable means that both layers of adhesive material will be moulded at the same time when the user applies finger pressure and thereby the user can manipulate the shape and contour of the barrier ring. In embodiments, the co-deformable adhesive material layers of the barrier ring can be stretched in the sense that the user is able to for example insert the index finger of each hand through the stoma-receiving opening of the barrier ring and provide a pulling force in opposite directions to thereby modify the size and/or shape of the barrier ring and/or the shape of the inner peripheral edge of the barrier ring. In examples, the barrier ring and the stoma-receiving opening are both generally annular or circular shaped when the barrier ring is supplied to the user before any manipulation or stretching, and subsequently takes an oval or elliptical shape after modification by the user.

Alternatively or additionally, in embodiments the user can subject the barrier ring to pressure, e.g. by placing a finger on opposite locations on the outer peripheral edge of the barrier ring and provide a pressure towards the stoma-receiving opening to thereby modify the shape of the barrier ring and/or the shape of the inner peripheral edge of the barrier ring. Thereby, the adhesive material layers of the ring will deform and displace adhesive material radially inwardly towards the opening and lead to a change in the shape of the barrier ring. The user is thus able to displace adhesive material from one portion of the barrier ring to another, which means that the second relatively softer adhesive material can be moved to form a thicker portion of the ring at a particular location corresponding to a position where a recession or skin fold around the user's stoma needs to be filled up by adhesive material for the creation of a tight fit or, conversely, form a thinner portion at another location. Particularly, but not exclusively, it is foreseen that the user can shape the pliable skin barrier ring from a generally annular or circular shape to a generally oval or elliptical shape. In embodiments, when the central portion of the barrier ring is manipulated by a user, a thickness of the barrier ring diminishes or increases because the materials of the first and second adhesive layers of the barrier ring are displaced radially away from or towards the stoma-receiving opening. Moreover, with the barrier ring according to the disclosure, the first adhesive material provides for the customized contour of the barrier ring, as obtained after a moulding operation by the user, to maintain its customized shape without any substantial elastic recovery to the original shape. This in combination with the second adhesive material's ability to flow more easily into the skin folds around the stoma, provides for a very versatile, pliable skin barrier ring. It has been found, by subjecting prototypes of the barrier ring described herein to tensile testing and comparing the results with the results of identical testing to an exemplary barrier ring of the prior art, that the pliable barrier ring of the disclosure exhibits significantly more plastic deformation capability than the prior art ring.

In embodiments, the barrier ring is configured to allow a maximum reduction in a thickness of the central portion of the ring of up to approximately 2 mm resulting from a single-axis-direction stretching of the ring at the inner peripheral edge (inner diameter of the stoma-receiving opening) of approximately 5 mm +/- 1 mm. In embodiments, the thickness of the barrier ring is diminished by up to 2 mm when the ring has undergone a resulting stretching of up to 6 mm as a result of an extension of the ring in opposite directions at the ring opening, whereby the shape of the barrier ring may e.g. shift from annular/circular to oval/elliptical. In embodiments, the barrier ring is configured to allow a maximum reduction in a thickness of the central portion of the ring of up to approximately 1 mm in response to a single-axis-direction stretching of the ring at the inner peripheral edge (inner diameter of the stoma-receiving opening) of approximately 5 mm +/- 1 mm. In embodiments, the barrier ring is configured to allow a maximum reduction in a thickness of the central portion of the ring of approximately 0.5 mm in response to the stretching. In embodiments, the barrier ring is configured to allow a maximum reduction in a thickness of the central portion of the ring of between approximately 0.5 - 1 mm in response to the stretching. In embodiments, the barrier ring is configured to diminish minimally in thickness in response to a stretching of up to 6 mm along one axis in a radial direction. Thus, a thickness of the central portion of the barrier ring according to the disclosure can be reduced by up to approximately 2 mm when the ring has undergone a resulting stretching along one axis in a radial direction. Any and all reductions in thickness between approximately 0.1 mm and approximately 2 mm, caused by a resulting stretching of up to approximately 6 mm along one axis of a radial direction of the barrier ring, are foreseen included in this disclosure.

When evaluating mouldability and/or tack/flow into the skin surface of adhesive materials in more specific terms, the parameters complex shear modulus |G*| and the tan(δ) within a frequency range can be used as good indicators of when an adhesive material is mouldable and/or tacky/flowable. These parameters are typically determined by deforming the adhesive material under rotational shear deformation to 1% controlled deformation at 32°C by using an oscillating frequency sweep.

The frequency range of interest is from 0,03 Hz - 1 Hz as this is the frequency range in which handling of ostomy devices, such as application, wear and removal typically occurs.

The complex shear modulus |G*| indicates the moulding characteristics of the adhesive material, i.e. how easy it is to shape and manipulate it with for example your fingers.

The tan(δ) of an adhesive material is a relation between the adhesive material's elastic properties and its plastic properties. The higher the elastic properties of an adhesive material, the more closely it will return to its original shape after it has been deformed, whereas if the plastic properties are high the adhesive material will remain closer to its deformed state. When tan(δ) = 1 the elastic and plastic properties are balanced. If tan(δ) is above 1, then the plastic properties are predominant and if tan(δ) is below 1 then the elastic properties are predominant. The adhesive materials of the present disclosure are considered mouldable if tan(δ) is above 0.6 at 1.0 Hz and |G*| is below 1 MPa in the frequency range of 0.03 - 1 Hz.

In embodiments, the relatively softer material of the second adhesive layer has a tangent delta (tan δ) above 0.6 and a |G*| below 1 MPa in a frequency range of 0.03 - 1.0 Hz with tangent delta at 0.03 Hz being below tangent delta at 1.0 Hz.

In embodiments, |G*| of the second relatively softer adhesive material is below |G*| of the first relatively harder adhesive material in a frequency range of 0.03 Hz - 1.0 Hz.

In embodiments, a difference in the respective |G*| of the first and second adhesive materials at a frequency of 0.03 Hz is at least 10 kPa and a difference of the respective |G*| of the first and second adhesive materials at a frequency of 1.0 Hz is at least 100 kPa.

Based on 122 responses from health care professionals in the field of ostomy care, being asked to compare a sample of the convex barrier ring according to the disclosure to a "standard-of-care" product available on the markets, i.e. Hollister^{®} Adapt Convex Barrier Ring, 94% of respondents found the barrier ring according to the disclosure to be better at providing convexity; 83% found it to be more skin friendly; 95% found it to have a better fit to individual body profiles; 98% found it to provide a better seal against the skin and 77% found it to be more easy and intuitive to use.

In a second aspect, the disclosure relates to an ostomy appliance system including a pliable skin barrier ring as disclosed herein, a base plate of the ostomy appliance and optionally a collecting bag for the ostomy appliance. The collecting bag is configured to be attached to a distal surface of the base plate. In one embodiment, the collecting bag is permanently attached to the base plate by welding the materials to each other. In other embodiments, each of the collecting bag and the base plate comprises a coupling half, each of which is configured to couple and engage in a liquid-tight manner with the other coupling half for attachment of the collecting bag to the base plate. In embodiments, a proximal surface of the base plate is adapted to be attached to a distal surface of the pliable skin barrier ring. In embodiments, the base plate includes a planar proximal surface. In other embodiments, the base plate includes a curved proximal surface. In an embodiment, the base plate is curved and invertible and provided with radially extending petals along an edge portion of the base plate, whereby the base plate is adaptable to be attached to a skin surface of a user around a stoma located on a skin bulge or hernia. In embodiments, the base plate is of the kind disclosed in applicant's publication WO 2016/146135.

Thereby, in embodiments according to the second aspect, a distal surface of the barrier ring is attached to the proximal surface of the base plate and thus configured to adhere to the peristomal skin surface immediately adjacent the stoma. In combination with the base plate, the convexity of the barrier ring helps provide for the ring 20 to make a recessed or retracted stoma better protrude from the skin surface due to the increased pressure immediately adjacent the stoma. In implementations wherein the ring is attached to the proximal surface of an adhesive wafer of an ostomy device, the expanded shape provides an additional pressure to the peristomal skin surface, because of the larger total adhesive area (attached to the abdominal skin surface) of the combined ostomy device.

### Dynamic mechanical analysis (DMA) - test conditions

The DMA was carried out as follows by a frequency sweep at 32°C and 1% CD (controlled deformation). Samples were prepared by thermoforming at 90°C, 10bar between two release liners to a thickness of 1 mm. With a punching tool, a round sample of either 25 mm or 8 mm in diameter was cut out (depending on test equipment dimensions). The release liners were removed, and the samples placed in a Thermo Scientific Haake Rheo Stress 6000 rheometer. The geometry applied was parallel plates with a diameter of 8 mm for the first relatively harder adhesive and 25 mm for the second relatively softer adhesive and the force applied was 5 N. After applying 5 N force, 10 minutes of thermal equilibration of the samples at 32°C were carried out before the measurements.

### Test procedure for stretching and measuring thickness of the barrier ring

The following description of a test procedure for carrying out a stretching of the barrier ring and for measurements of the thickness of the barrier before and after the stretching is illustrative and should be considered one possible way of doing the stretching and determining the thickness. Other methods and procedures for stretching the barrier ring and measuring its thickness can be acceptable.

In the test procedure for the barrier ring according to the disclosure a customized stretching tool was prepared. However, this is not essential for obtaining the stretching of the barrier ring and will therefore not be described further.

The steps of the testing procedure are as follows:
1. Prepare a barrier ring sample
2. Draw four lines uniquely from the top of the convex side (proximally projecting expanded shape of the ring) and into the adhesive material in the stoma-receiving opening, such that two opposite lines are aligned and that the two pairs of lines are perpendicular (orthogonal) to each other
3. Measure the thickness of the barrier ring at the convex side end of each of the four lines (see below for measuring procedure) and mark each point uniquely, e.g. "1", "2", "3", "4"
4. Measure the inner diameter of the barrier ring (diameter of stoma-receiving opening) between two of the oppositely aligned lines made in step 2 with either a ruler or a calliper
5. Place the sample with the convex side facing up and with two of the oppositely marked points (e.g. points "1" and "3") in line with the stretching direction
6. Grip the sample at the inner periphery by the oppositely marked points and subject the barrier ring sample to oppositely directed pulling forces along the axis of the oppositely aligned lines and the size of the opening by 15 mm
7. Maintain the stretched state of the sample for 40 +/- 10 seconds
8. Release tension on the sample and place it on a low-resistance surface with the convex side facing up and let it settle freely
9. Let the sample dwell for 30 +/- 2 minutes to allow it to retract from the stretched condition
10. Repeat step 4 to measure the inner diameter after the stretching
11. Measure the resulting thickness of the barrier ring at the convex side at each of the four uniquely marked points
12. Compare the thickness measurements after the stretching of the barrier ring in step 11 with the thickness measurements before the stretching of the barrier ring in step 4

The thickness of the barrier ring is measured as follows:
1. A calibrated thickness gauge is provided, such as "Absolute" from Mitutoyo. The thickness gauge should have an accuracy of 0.02 mm or better and a measuring force of 1.5 N or less. The measuring foot is preferably 10 mm in diameter with a planar surface.
2. A sample of the barrier ring is provided
3. The thickness gauge is reset by doing a measurement with no sample between the measuring foot and the gauge plane
4. One of the marked points of the barrier ring sample is placed between the measuring foot and the gauge plane and a measurement is recorded
5. Step 4 is repeated at all the marked points on the barrier ring sample

For some of the testing procedures and the measurements, it may be helpful for practical purposes if one or more of the surfaces of the adhesive materials of the barrier ring are covered with a release liner material, e.g. a siliconized polymeric sheet material. The skilled person will be able to determine the need for this and apply such a material, if considered needed. Similarly, the skilled person will have no difficulty in taking due account of the additional thickness of the release liner(s) if applied.

### Detailed description of the drawings

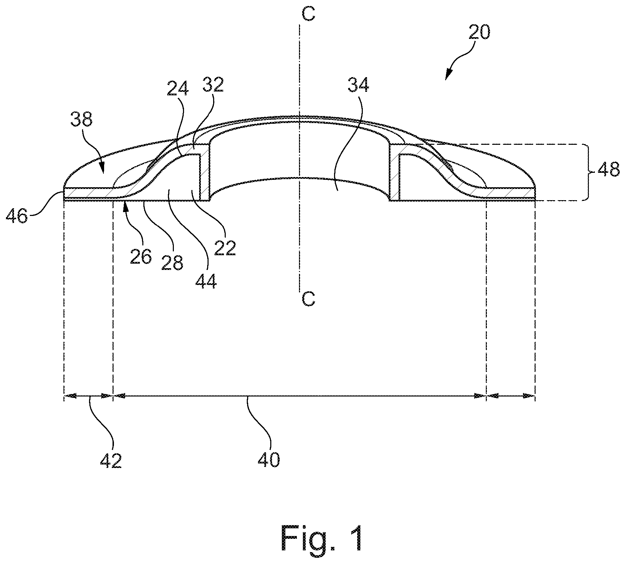

Figure 1 is a cross-sectional, perspective view of one embodiment of a pliable barrier ring 20 according to the disclosure.

The pliable barrier ring 20 includes a first layer of a relatively harder adhesive material 22 having a proximal surface 24 and a distal surface 26. The distal surface 26 of the first adhesive layer 22 forms at least a portion of a plane (planar) distal surface 28 of the barrier ring 20. The ring 20 further includes a second layer of a relatively softer adhesive material 32 provided at least on the proximal surface 24 of the first layer of adhesive material 22. The second layer of adhesive material 32 forms a proximal surface 38 of the ring 20. The ring 20 includes a stoma-receiving opening 34 extending through a central portion 40. The first layer of adhesive material 22 includes a relatively thicker bulk portion 44 located in the central portion 40 of the ring 20, and a relatively thinner flange portion 42 extending radially inwards from an outer peripheral edge 46 of the ring 20. The flange portion 42 transitions into the bulk portion 44. The relatively thicker bulk portion 44 provides a proximally projecting expanded shape 48 to the central portion 40 of the ring 20. The proximally projecting expanded shape 48 incurred by the bulk portion 44 can be understood to give the ring 20 an overall convex shape, as explained above.

The pliable skin barrier ring 20 is adapted for placement in a skin fold or crease located around a stoma of a user. The ring 20 is configured to adhere to the peristomal skin surface immediately adjacent the stoma and is used for providing a seal around the stoma and will in many implementations be used in combination with a regular ostomy device comprising a collecting pouch and an adhesive wafer. Particularly, the expanded shape 48 incurs a convexity to the ring 20, which helps provide for the ring 20 to make a recessed or retracted stoma better protrude from the skin surface due to the increased pressure immediately adjacent the stoma. In some implementations wherein the ring 20 is attached to the proximal surface of an adhesive wafer of an ostomy device, the expanded shape 48 may provide an additional pressure to the peristomal skin surface, because of the larger total adhesive area (attached to the abdominal skin surface) of the combined ostomy device.

The first layer of relatively harder adhesive material 22, with its bulk portion 44, provides enough dimensional stability to the pliable barrier ring 20 to incur the required pressure to the skin surface around the stoma. The first relatively harder adhesive material 22, however, also exhibits mouldability to allow the user to customize the ring 20. The second layer of relatively softer adhesive material 32 is configured not only to be moulded in combination with the first adhesive material 22, it is further adapted to begin flowing at a lower temperature than the second adhesive material 32, and therefore it suitably flows into folds and creases of the skin surface around the recessed stoma before the first adhesive material 22. The combination of the two, differentiated adhesive layers 22, 32 in a peristomal pressure incurring pliable barrier ring, being dimensionally stable, yet customizable (mouldable) and with the ability to create a good seal around a recessed stoma, to a larger extent meets the users' need for a better seal around the stoma, particularly, but not exclusively, for users with outwardly protruding body profiles.

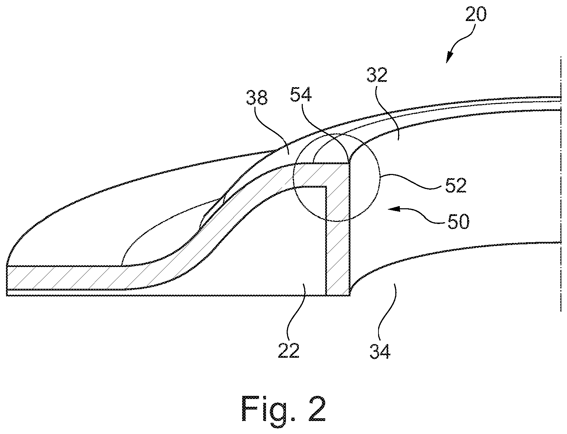

Figure 2 is an enlarged cross-sectional, perspective partial view of one embodiment of a pliable barrier ring 20 according to the disclosure.

In Fig. 2, the second layer of relatively softer adhesive material 32 forms an entirety of an inner peripheral surface 50 of the ring 20 at the stoma-receiving opening 34. In embodiments, none of the material 22 of the first adhesive layer abuts or forms an interface with the stoma-receiving opening 34. In the embodiment illustrated in Fig.2, the inner peripheral surface 50 annularly surrounds the stoma-receiving opening 34. In Fig. 2, the second layer of the relatively softer adhesive material 32 provides a "lining" of the entire extent of the inner peripheral surface 50 of the ring 20 at the stoma-receiving opening 34. The embodiment illustrated in the view of Fig. 2 further includes a transition 52 between the proximal surface 38 of the barrier ring 20 and the inner peripheral surface 50 of the ring 20 at the stoma-receiving opening 34, both surfaces 38, 50 formed by the second layer of relatively softer adhesive material 32. In Fig. 2, the transition 52 includes an edge 54 where a plane of the proximal surface 38 of the barrier ring 20 at the central portion 40 meets a tangent to the inner peripheral surface 50 of the ring 20 at the stoma-receiving opening in a substantially orthogonal or perpendicular manner.

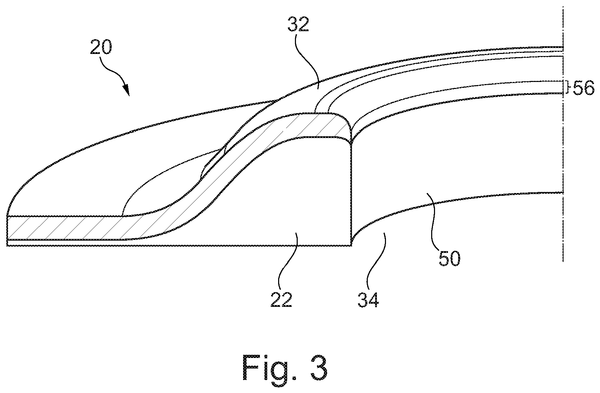

Figure 3 is an enlarged cross-sectional, perspective partial view of one embodiment of a pliable barrier ring 20 according to the disclosure.

In Fig. 3, the first layer of relatively harder adhesive material 22 forms a major portion of the inner peripheral surface 50 of the ring 20 at the stoma-receiving opening 34. In embodiments, the material 22 of the first adhesive layer abuts or forms an interface with the stoma-receiving opening 34. In the embodiment illustrated in Fig.3, the inner peripheral surface 50 annularly surrounds the stoma-receiving opening 34. In embodiments, also a minor edge portion 56 of the second layer of relatively softer adhesive material 32 abuts or forms an interface with the stoma-receiving opening 34.

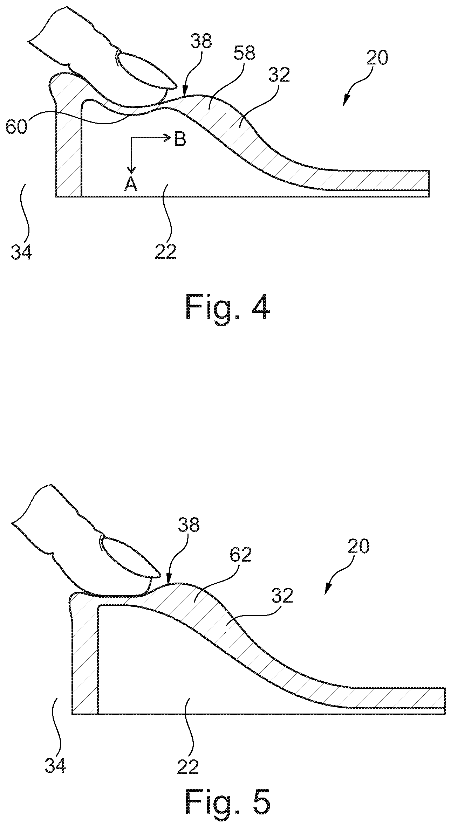

Figure 4 is a schematic, cross-sectional view of one embodiment of a pliable barrier ring 20 according to the disclosure.

In Fig. 4, both the second relatively softer adhesive material layer 32 and the first layer of relatively harder adhesive material 22 are seen to be deformable by finger pressure applied by a user, i.e. the first and second layers of adhesive material 22, 32 are co-deformable. When the user applies a finger pressure, the shape and/or contour of the barrier ring 20 is manipulated. This co-deformability of the adhesive materials 22, 32 is illustrated in the embodiment of Fig. 4. Firstly, in that a portion (or volume) of the second softer adhesive material layer 32 is displaced radially outward in relation to the stoma-receiving opening 34 to form a local bulge 58 of the second adhesive material, which is thicker than the adjacent portions of the second adhesive material 32. It should be understood that the second adhesive material 32 is not necessarily only displaced in a radially outward direction, but also potentially in directions crossing the radial directions, i.e. "sideways", and radially inward. Secondly, also in that the view of the embodiment of Fig. 4 further illustrates the first layer of relatively harder adhesive material 22 being deformed axially and radially in the direction of the arrows A and B. Additionally, in Fig. 4 a slight depression 60 in the first adhesive material 22 can be seen where the user has put pressure on the proximal surface 38 of the ring. It is to be understood that the deformation of both adhesive materials 22, 32 in these embodiments happens simultaneously. It is further to be understood that the user can displace adhesive material 22, 32 from one portion (segment/section) of the barrier ring 20 to another, which means that adhesive material 22, 32 can be moved to form a thicker portion 58 of the ring at a specific location, as illustrated in Fig. 4. A resulting customized contour of the barrier ring 20, as obtained after the finger moulding by the user, provides for an improved fit of the customized barrier ring 20 to the skin surface contour around the stoma.

Figure 5 is a schematic, cross-sectional view of one embodiment of a pliable barrier ring 20 according to the disclosure.

In the embodiment of Fig. 5, the second relatively softer adhesive material layer 32 is deformable by the finger pressure applied by a user. When the user applies a finger pressure to the proximal surface 38 of the ring 20, the shape and/or contour of the barrier ring 20 is manipulated (i.e. in these embodiments, of the proximal surface 38 of the ring). Fig. 5 illustrates how a portion (or volume) of the second softer adhesive material layer 32 is displaced radially outward in relation to the stoma-receiving opening 34 to form a local bulge 62 of the second adhesive material, which is thicker than adjacent portions of the second adhesive material 32. It should be understood that the second adhesive material 32 is not necessarily only displaced in a radially outward direction, but also potentially in directions crossing the radial directions, i.e. "sideways", and radially inward.

Notably, in the view of the embodiment of Fig. 5, the first layer of relatively harder adhesive material 22 is not deformed by the user's application of finger pressure. It is to be understood that in the embodiments of Fig. 5, the differentiation between the relatively harder first adhesive 22 and the relatively softer second adhesive 22 provides for these characteristics. In other words, the first and second adhesive materials 22, 32 are configured such that no, or only insubstantial, simultaneous deformation of the materials takes place. The user can therefore displace the second relatively softer adhesive material 32 from one portion (segment/section) of the barrier ring 20 to another, which means that the second softer adhesive material 32 can be moved to form a thicker portion 62 of the ring at a specific location, while at the same time the first relatively harder adhesive material 22 maintains its shape (proximally projecting portion) without compromising the overall dimensional stability of the ring 20, as illustrated in Fig. 5. A resulting customized contour of the barrier ring 20, as obtained after the finger moulding of the second softer adhesive layer 32 by the user, provides for a barrier ring 20 both offering great versatility in terms of adaptation to the skin surface contour and hence an improved fit around the stoma, but which also maintains sufficient pressure in the peristomal area to keep a recessed stoma protruding from the skin surface.

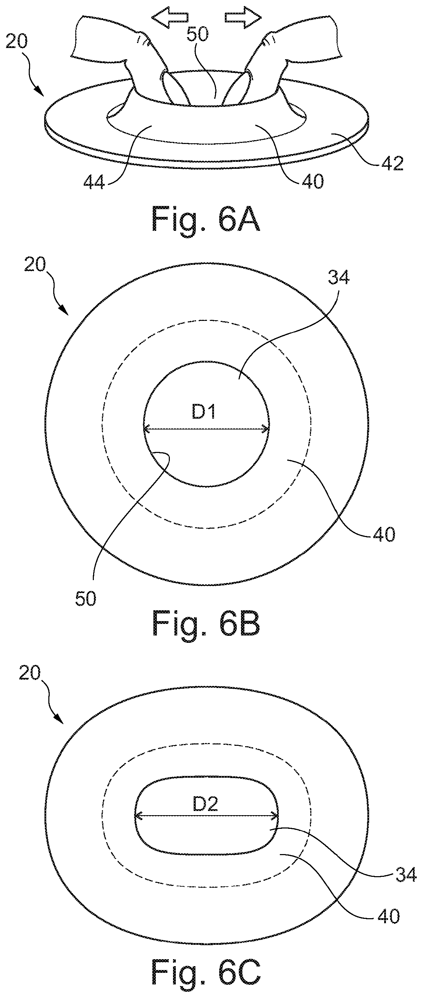

Figure 6A is a schematic, perspective view of one embodiment of a pliable barrier ring 20 of the disclosure showing the ring being stretched by the fingers of a user. Figure 6B is a schematic top view of the pliable barrier ring 20 of Figure 6A before the ring has been stretched. Figure 6C is a schematic top view of the pliable barrier ring 20 of Figures 6A, 6B after the ring 20 has been stretched. Referring first to Figure 6A, in embodiments, a thickness of the central portion 40 of the pliable barrier ring 20 (compare reference number 48 in Figure 1 indicating the proximally projecting expanded shape of the central portion 40) is reduced when stretching the ring at the inner peripheral surface 50 (inner diameter D1) of the stoma-receiving opening 34, cf. also Figures 6B and 6C. In other words, in embodiments the thickness of the pliable barrier ring 20 is reduced when the ring 20 has undergone a stretching as the result of the extension of the ring in opposite directions at the opening 34, whereby the shape of the pliable barrier ring 20 can be seen to have shifted from annular/circular in Figure 6B to oval/elliptical in Figure 6C. Also indicated in Figure 6A is the bulk portion 44 and the flange portion 42. Comparing the views of Figures 6B and 6C, it can also be seen how a diameter of the opening 34 has been enlarged from an initial diameter D1 in Figure 6B to a greater post-stretching diameter D2 in Figure 6C. Notably, the deformation of the ring 20, schematically indicated in Figure 6C, is permanent. In embodiments, a reduction in thickness of the central portion 40 of the barrier ring 20 is maximally 2 mm when the ring 20 has been stretched in the axial direction of the arrows corresponding to diameter D2 in Figure 6C and subsequently allowed to settle.

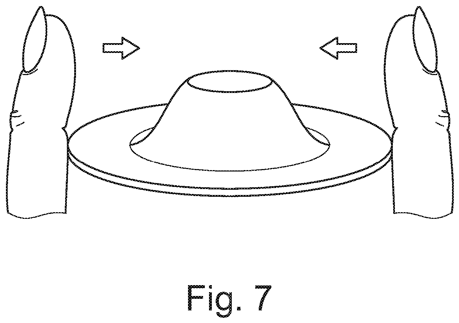

Figure 7 is a schematic, perspective view of one embodiment of a pliable barrier ring 20 of the disclosure showing the ring being compressed between fingers of a user.

Although particular features have been shown and described, it will be understood that they are not intended to limit the claimed invention, and it will be made obvious to those skilled in the art that various changes and modifications may be made without departing from the scope of the claimed invention. The specification and drawings are, accordingly, to be regarded in an illustrative rather than restrictive sense. Therefore, it is intended that this invention be limited only by the claims and the equivalents thereof.

## Claims

1. A pliable skin barrier ring (20) adapted for placement in a skin fold or crease located around a stoma of a user, the pliable barrier ring (20) comprising:
a first adhesive layer of a first adhesive material (22), the first adhesive layer having a proximal surface (24) configured to face the skin of the user during use and a distal surface (26) configured to face away from the skin of the user during use, the distal surface (26) of the first adhesive layer (22) forming at least a portion of a plane distal surface (28) of the skin barrier ring (20);
a second adhesive layer of a second adhesive material (32), the second adhesive material (32) being relatively softer than the first adhesive material (22), the second adhesive layer (32) being provided at least on the proximal surface (24) of the first adhesive layer (22), the second adhesive layer (32) forming a proximal surface (38) of the skin barrier ring (20);
a stoma-receiving opening (34) extending through a central portion (40) of the skin barrier ring (20);
wherein the first adhesive layer (22) comprises a bulk portion (44) located in the central portion (40) of the skin barrier ring, and a flange portion (42) being thinner than the bulk portion (44), the flange portion (42) extending radially inwards from an outer peripheral edge (46) of the skin barrier ring (20) and transitioning into the bulk portion (44); and
wherein the bulk portion (44) provides a proximally projecting expanded shape (48) to the central portion (40) of the skin barrier ring (20).

2. The pliable skin barrier ring of claim 1, wherein the second adhesive layer (32) forms an entirety of an inner peripheral surface (50) of the skin barrier ring (20) at the stoma-receiving opening (34).

3. The pliable skin barrier ring of claim 1, wherein the proximal surface (38) of the skin barrier ring (20) describes a curve approximating an S-shape, when seen in a cross-sectional view through the skin barrier ring.

4. The pliable skin barrier ring of claim 1, wherein a first portion of the proximal surface (38) of the skin barrier ring (20) provided on the flange portion (42) of the first adhesive layer (22) lies in a first plane, and wherein a second portion of the proximal surface (38) of the skin barrier ring (20) provided on the bulk portion (44) of the first adhesive layer (22) lies in a second plane, and wherein the second plane is parallel to the first plane.

5. The pliable skin barrier ring (20) of claim 4, wherein a linear distance between the first plane and the second plane measured along a normal to the first or the second plane is in the range of 5-20 mm.

6. The pliable skin barrier ring of claim 1, wherein the second adhesive layer (32) is configured to have a substantially constant thickness measured in an axial direction of the skin barrier ring (20).

7. The pliable skin barrier ring of claim 1, wherein the substantially constant thickness of the second adhesive layer (32) is selected from a range of 0.5 - 10.0 mm.

8. The pliable skin barrier ring of claim 1, wherein a thickness of the flange portion (42) of the first adhesive layer (22) measured in an axial direction of the skin barrier ring (20) is in a range of 0.2 - 3.0 mm.

9. The pliable skin barrier ring of claim 1, wherein a maximum thickness of the bulk portion (44) of the first layer of adhesive material (22) measured in an axial direction of the skin barrier ring (20) is in a range of 3 - 12 mm, such as 3 - 10 mm.

10. The pliable skin barrier ring (20) of any one of the preceding claims, wherein the first adhesive material (22) has a tangent delta (tan δ) below 0.6 at 1.0 Hz and a |G*| below 1 MPa in a frequency range of 0.03 - 1.0 Hz with tangent delta at 0.03 Hz being above tangent delta at 1.0 Hz.

11. The pliable skin barrier ring (20) of any one of the preceding claims, wherein the second adhesive material (32) has a tangent delta (tan δ) above 0.6 and a |G*| below 1 MPa in a frequency range of 0.03 - 1.0 Hz with tangent delta at 0.03 Hz being below tangent delta at 1.0 Hz.

12. The pliable skin barrier ring (20) of claim 1, wherein |G*| of the second adhesive material (32) is below |G*| of the first adhesive material (22) in a frequency range of 0.03 Hz - 1.0 Hz.

13. The pliable skin barrier ring (20) of claim 1, wherein a difference in the respective |G*| of the first adhesive material (22) and the second adhesive material (32) at a frequency of 0.03 Hz is at least 10 kPa and wherein a difference of the respective |G*| of the first and second adhesive materials (22, 32) at a frequency of 1.0 Hz is at least 100 kPa.

14. The pliable skin barrier ring (20) of claim 1, wherein the second adhesive layer (32) can be displaced on the proximal surface (24) of the first adhesive layer (22) without simultaneously deforming the proximal surface of the first adhesive layer (22).

15. An ostomy appliance system, comprising:
a pliable skin barrier ring (20) according to any one of claims 1-14;
a base plate of the ostomy appliance; and, optionally,
a collecting bag for the ostomy appliance configured to be attached to a distal surface of the base plate;
wherein a proximal surface of the base plate is adapted to be attached to a distal surface of the pliable skin barrier ring (20).

16. The ostomy appliance system of claim 15, wherein the base plate is adapted to be attached to a skin surface of a user around a stoma located on a skin bulge or hernia, wherein the base plate is curved and invertible and provided with radially extending petals along an edge portion of the base plate.

## Patentansprüche

1. Biegsamer Hautbarrierenring (20), ausgelegt zur Platzierung in einer Hautfalte oder Falte, die um ein Stoma eines Benutzers angeordnet ist, wobei der biegsame Barrierenring (20) umfasst:
eine erste Klebstoffschicht aus einem ersten Klebstoffmaterial (22), wobei die erste Klebstoffschicht eine proximale Oberfläche (24), die dafür gestaltet ist, in Verwendung der Haut des Benutzers zugewandt zu sein, und eine distale Oberfläche (26), die dafür gestaltet ist, in Verwendung von der Haut des Benutzers weg zu weisen, aufweist, wobei die distale Oberfläche (26) der ersten Klebstoffschicht (22) wenigstens einen Abschnitt einer ebenen distalen Oberfläche (28) des Hautbarrierenrings (20) bildet;
eine zweite Klebstoffschicht aus einem zweiten Klebstoffmaterial (32), wobei das zweite Klebstoffmaterial (32) relativ weicher als das erste Klebstoffmaterial (22) ist, wobei die zweite Klebstoffschicht (32) wenigstens auf der proximalen Oberfläche (24) der ersten Klebstoffschicht (22) bereitgestellt ist, wobei die zweite Klebstoffschicht (32) eine proximale Oberfläche (38) des Hautbarrierenrings (20) bildet;
eine Stoma-aufnehmende Öffnung (34), die durch einen zentralen Abschnitt (40) des Hautbarrierenrings (20) verläuft;
wobei die erste Klebstoffschicht (22) einen Volumenabschnitt (44), der in dem zentralen Abschnitt (40) des Hautbarrierenrings angeordnet ist, und einen Flanschabschnitt (42), der dünner als der Volumenabschnitt (44) ist, umfasst, wobei der Flanschabschnitt (42) von einer äußeren Umfangskante (46) des Hautbarrierenrings (20) radial nach innen verläuft und in den Volumenabschnitt (44) übergeht; und
wobei der Volumenabschnitt (44) eine proximal vorstehende expandierte Form (48) für den zentralen Abschnitt (40) des Hautbarrierenrings (20) bereitstellt.

2. Biegsamer Hautbarrierenring nach Anspruch 1, wobei die zweite Klebstoffschicht (32) eine Gesamtheit einer inneren Umfangsfläche (50) des Hautbarrierenrings (20) an der Stoma-aufnehmenden Öffnung (34) bildet.

3. Biegsamer Hautbarrierenring nach Anspruch 1, wobei die proximale Oberfläche (38) des Hautbarrierenrings (20) eine Kurve beschreibt, die, wenn in einer Querschnittsansicht durch den Hautbarrierenring gesehen, etwa S-förmig ist.

4. Biegsamer Hautbarrierenring nach Anspruch 1, wobei ein erster Abschnitt der proximalen Oberfläche (38) des Hautbarrierenrings (20), der an dem Flanschabschnitt (42) der ersten Klebstoffschicht (22) bereitgestellt ist, in einer ersten Ebene liegt, und wobei ein zweiter Abschnitt der proximalen Oberfläche (38) des Hautbarrierenrings (20), der an dem Volumenabschnitt (44) der ersten Klebstoffschicht (22) bereitgestellt ist, in einer zweiten Ebene liegt, und wobei die zweite Ebene parallel zu der ersten Ebene ist.

5. Biegsamer Hautbarrierenring (20) nach Anspruch 4, wobei ein linearer Abstand zwischen der ersten Ebene und der zweiten Ebene, gemessen entlang einer Normalen zu der ersten oder der zweiten Ebene, in dem Bereich von 5-20 mm liegt.

6. Biegsamer Hautbarrierenring nach Anspruch 1, wobei die zweite Klebstoffschicht (32) dafür gestaltet ist, eine im Wesentlichen konstante Dicke, gemessen in einer axialen Richtung des Hautbarrierenrings (20), aufzuweisen.

7. Biegsamer Hautbarrierenring nach Anspruch 1, wobei die im Wesentlichen konstante Dicke der zweiten Klebstoffschicht (32) ausgewählt ist aus einem Bereich von 0,5-10,0 mm.

8. Biegsamer Hautbarrierenring nach Anspruch 1, wobei eine Dicke des Flanschabschnitts (42) der ersten Klebstoffschicht (22), gemessen in einer axialen Richtung des Hautbarrierenrings (20), in einem Bereich von 0,2-3,0 mm liegt.

9. Biegsamer Hautbarrierenring nach Anspruch 1, wobei eine maximale Dicke des Volumenabschnitts (44) der ersten Schicht von Klebstoffmaterial (22), gemessen in einer axialen Richtung des Hautbarrierenrings (20), in einem Bereich von 3-12 mm, wie z.B. 3-10 mm, liegt.

10. Biegsamer Hautbarrierenring (20) nach einem der vorstehenden Ansprüche, wobei das erste Klebstoffmaterial (22) ein tangens delta (tan δ) unter 0,6 bei 1,0 Hz und ein |G*| unter 1 MPa in einem Frequenzbereich von 0,03-1,0 Hz aufweist, wobei tangens delta bei 0,03 Hz über tangens delta bei 1,0 Hz liegt.

11. Biegsamer Hautbarrierenring (20) nach einem der vorstehenden Ansprüche, wobei das zweite Klebstoffmaterial (32) ein tangens delta (tan δ) über 0,6 und ein |G*| unter 1 MPa in einem Frequenzbereich von 0,03-1,0 Hz aufweist, wobei tangens delta bei 0,03 Hz unter tangens delta bei 1,0 Hz liegt.

12. Biegsamer Hautbarrierenring (20) nach Anspruch 1, wobei |G*| des zweiten Klebstoffmaterials (32) in einem Frequenzbereich von 0,03 Hz bis 1,0 Hz unter |G*| des ersten Klebstoffmaterials (22) liegt.

13. Biegsamer Hautbarrierenring (20) nach Anspruch 1, wobei eine Differenz des entsprechenden |G*| des ersten Klebstoffmaterials (22) und des zweiten Klebstoffmaterials (32) bei einer Frequenz von 0,03 Hz wenigstens 10 kPa beträgt und wobei eine Differenz des entsprechenden |G*| des ersten und des zweiten Klebstoffmaterials (22, 32) bei einer Frequenz von 1,0 Hz wenigstens 100 kPa beträgt.

14. Biegsamer Hautbarrierenring (20) nach Anspruch 1, wobei die zweite Klebstoffschicht (32) auf der proximalen Oberfläche (24) der ersten Klebstoffschicht (22) verschoben werden kann, ohne gleichzeitig die proximale Oberfläche der ersten Klebstoffschicht (22) zu verformen.

15. Stomavorrichtungssystem umfassend:
einen biegsamen Hautbarrierenring (20) nach einem der Ansprüche 1-14;
eine Basisplatte der Stomavorrichtung; und
gegebenenfalls
einen Sammelbeutel für die Stomavorrichtung, dafür gestaltet, an einer distalen Oberfläche der Basisplatte angebracht zu werden;
wobei eine proximale Oberfläche der Basisplatte dafür ausgelegt ist, an einer distalen Oberfläche des biegsamen Hautbarrierenrings (20) angebracht zu werden.

16. Stomavorrichtungssystem nach Anspruch 15, wobei die Basisplatte dafür ausgelegt ist, an einer Hautoberfläche eines Benutzers um ein Stoma angebracht zu werden, das an einer Hautausbuchtung oder Hernie angeordnet ist, wobei die Basisplatte gekrümmt und umwendbar ist und mit radial verlaufenden Blättern entlang eines Randabschnitts der Basisplatte versehen ist.

## Revendications

1. Anneau de barrière cutanée pliable (20) conçu pour être placé dans un pli ou repli cutané situé autour de la stomie d'un utilisateur, l'anneau de barrière pliable (20) comprenant :
une première couche adhésive d'un premier matériau adhésif (22), la première couche adhésive ayant une surface proximale (24) configurée pour faire face à la peau de l'utilisateur lors de l'utilisation et une surface distale (26) configurée pour être tournée à l'opposé de la peau de l'utilisateur lors de l'utilisation, la surface distale (26) de la première couche adhésive (22) formant au moins une partie d'une surface distale plane (28) de l'anneau de barrière cutanée (20) ;
une seconde couche adhésive d'un second matériau adhésif (32), le second matériau adhésif (32) étant relativement plus mou que le premier matériau adhésif (22), la seconde couche adhésive (32) étant prévue au moins sur la surface proximale (24) de la première couche adhésive (22), la seconde couche adhésive (32) formant une surface proximale (38) de l'anneau de barrière cutanée (20) ;
une ouverture de réception de stomie (34) s'étendant à travers une partie centrale (40) de l'anneau de barrière cutanée (20) ;
la première couche adhésive (22) comprenant une partie massive (44) située dans la partie centrale (40) de l'anneau de barrière cutanée, et une partie de bride (42) étant plus mince que la partie massive (44), la partie de bride (42) s'étendant radialement vers l'intérieur à partir d'un bord périphérique extérieur (46) de l'anneau de barrière cutanée (20) et passant dans la partie massive (44) ; et
la partie massive (44) conférant une forme expansée faisant saillie de manière proximale (48) à la partie centrale (40) de l'anneau de barrière cutanée (20).

2. Anneau de barrière cutanée pliable selon la revendication 1, la seconde couche adhésive (32) formant la totalité d'une surface périphérique intérieure (50) de l'anneau de barrière cutanée (20) au niveau de l'ouverture de réception de stomie (34).

3. Anneau de barrière cutanée pliable selon la revendication 1, la surface proximale (38) de l'anneau de barrière cutanée (20) décrivant une courbe s'approchant d'une forme en S, lorsqu'on la voit dans une vue en coupe transversale à travers l'anneau de barrière cutanée.

4. Anneau de barrière cutanée pliable selon la revendication 1, une première partie de la surface proximale (38) de l'anneau de barrière cutanée (20) prévue sur la partie de bride (42) de la première couche adhésive (22) se situant dans un premier plan, et une seconde partie de la surface proximale (38) de l'anneau de barrière cutanée (20) prévue sur la partie massive (44) de la première couche adhésive (22) se situant dans un second plan, et le second plan étant parallèle au premier plan.

5. Anneau de barrière cutanée pliable (20) selon la revendication 4, une distance linéaire entre le premier plan et le second plan mesurée le long d'une normale au premier ou au second plan étant dans la plage de 5-20 mm.

6. Anneau de barrière cutanée pliable selon la revendication 1, la seconde couche adhésive (32) étant configurée pour avoir une épaisseur sensiblement constante mesurée dans une direction axiale de l'anneau de barrière cutanée (20).

7. Anneau de barrière cutanée pliable selon la revendication 1, l'épaisseur sensiblement constante de la seconde couche adhésive (32) étant choisie dans une plage de 0,5-10,0 mm.

8. Anneau de barrière cutanée pliable selon la revendication 1, une épaisseur de la partie de bride (42) de la première couche adhésive (22) mesurée dans une direction axiale de l'anneau de barrière cutanée (20) étant dans une plage de 0,2-3,0 mm.

9. Anneau de barrière cutanée pliable selon la revendication 1, une épaisseur maximale de la partie massive (44) de la première couche de matériau adhésif (22) mesurée dans une direction axiale de l'anneau de barrière cutanée (20) étant dans une plage de 3-12 mm, par exemple 3-10 mm.

10. Anneau de barrière cutanée pliable (20) selon l'une quelconque des revendications précédentes, le premier matériau adhésif (22) ayant une tangente delta (tan δ) inférieure à 0,6 à 1,0 Hz et une |G*| inférieure à 1 MPa dans une plage de fréquences de 0,03-1,0 Hz, la tangente delta à 0,03 Hz étant supérieure à la tangente delta à 1,0 Hz.

11. Anneau de barrière cutanée pliable (20) selon l'une quelconque des revendications précédentes, le second matériau adhésif (32) ayant une tangente delta (tan δ) supérieure à 0,6 et une |G*| inférieure à 1 MPa dans une plage de fréquences de 0,03-1,0 Hz, la tangente delta à 0,03 Hz étant inférieure à la tangente delta à 1,0 Hz.

12. Anneau de barrière cutanée pliable (20) selon la revendication 1, |G*| du second matériau adhésif (32) étant inférieur à |G*| du premier matériau adhésif (22) dans une plage de fréquences de 0,03 Hz-1,0 Hz.

13. Anneau de barrière cutanée pliable (20) selon la revendication 1, une différence dans le |G*| respectif du premier matériau adhésif (22) et du second matériau adhésif (32) à une fréquence de 0,03 Hz étant d'au moins 10 kPa et une différence du |G*| respectif des premier et second matériaux adhésifs (22, 32) à une fréquence de 1,0 Hz étant d'au moins 100 kPa.

14. Anneau de barrière cutanée pliable (20) selon la revendication 1, la seconde couche adhésive (32) pouvant être déplacée sur la surface proximale (24) de la première couche adhésive (22) sans déformer simultanément la surface proximale de la première couche adhésive (22).

15. Système d'appareil de stomie comprenant :
un anneau de barrière cutanée pliable (20) selon l'une quelconque des revendications 1-14 ;
une plaque de base de l'appareil de stomie ; et, éventuellement,
un sac collecteur pour l'appareil de stomie configuré pour être fixé à une surface distale de la plaque de base ;
une surface proximale de la plaque de base étant conçue pour être fixée à une surface distale de l'anneau de barrière cutanée pliable (20).

16. Système d'appareil de stomie selon la revendication 15, la plaque de base étant adaptée pour être fixée à une surface cutanée d'un utilisateur autour d'une stomie située sur un renflement cutané ou une hernie, la plaque de base étant incurvée et inversible et munie de pétales s'étendant radialement le long d'une partie de bord de la plaque de base.
